# EUROPEAN PATENT APPLICATION

(11) **EP 2 153 867 A2**
(43) Date of publication of application: **17.02.2010**
(21) Application number: 09167467.1
(22) Date of filing: 07.08.2009
(51) Int. Cl.: A61N 1/36

(54) **System and method for transvascular activation of cardiac nerves to improve heart function**

(30) Priority: 12.08.2008 US 190097
(71) Applicant: Curtis, Guy P., San Diego, CA 92110 (US)
(72) Inventor: Curtis, Guy P., San Diego, CA 92110 (US)
(74) Representative: Gates, Marie Christina Esther

(57) **Abstract**

A system for electrically stimulating the heart muscle to improve heart function requires identifying a site in the venous system adjacent a sympathetic nerve. An electrode is then positioned at the site to electrically stimulate the nerve. In turn, this stimulation releases norepinephrine from the nerve to improve heart muscle contraction.

## Description

### FIELD OF THE INVENTION

The present invention pertains generally to systems and methods for treating heart disease. More particularly, the present invention pertains to systems and methods for electrically stimulating the heart muscle to improve heart function. The present invention is particularly, but not exclusively, useful as a system or method wherein an electrode is positioned in the coronary sinus, or a vein connected with the coronary sinus, to be adjacent a sympathetic nerve for stimulation of the nerve and muscle to improve heart muscle contractions.

### BACKGROUND OF THE INVENTION

Normal heart function is characterized by a rhythmic contraction of the heart muscle, wherein each contraction is followed by a refractory period and cardiac diastole during which the heart muscle relaxes to be refilled with circulating blood. A diseased heart, however, may experience filling disorders or ineffective contractions under certain conditions that diminish heart and circulatory function leading to heart failure. In response to a diminished heart function, a relatively common practice has been to place electrodes via the cardiac veins on the epicardial surface of the heart's left ventricle, and to then electrically stimulate the area of placement for the purpose of synchronizing heart contractions. Such a technique, however, is not predictably effective and has resulted in no benefit in significant numbers of patients where substantial benefit would have been predicted. Moreover, the possibility of cell damage in the area of electrode placement from observed loss in contractile function has raised additional concerns.

Though several mechanisms are known to contribute to contractions of the heart muscle, they each do so in different ways. As indicated above, one such mechanism involves a direct electrical stimulation of the heart muscle and control of the sequence of muscle activation. Another mechanism for improving muscle function, however, involves the stimulation of sympathetic nerves. More specifically, it is known that norepinephrine (a derivative of adrenaline released from the nervous system nerve endings at the heart) is a potent stimulant of contraction on the heart muscle. It is also known that sympathetic nerves can be electrically stimulated to secrete norepinephrine in response to relatively low intensity stimulation patterns, Importantly, stimulation of the sympathetic nervous system can be efficacious for energizing the heart muscle by causing the release of norepinephrine at low stimulation intensities that do not have any direct effects to electrically stimulate the heart muscle itself.

In light of the above, it is an object of the present invention to provide a system and method that will improve heart function by indirectly stimulating the sympathetic nervous system. Another object of the present invention is to provide a system and method that avoids direct stimulation of the heart muscle while improving heart function with indirect electrical stimulation. Yet another object of the present invention is to improve heart function using low intensity, electrical stimulation patterns during the heart's refractory period that will not adversely affect the heart muscle, or otherwise diminish its local muscle function. Another object of the present invention is to provide a system and method for improving heart function by electrical stimulation that alters the sequence of muscle activation but is also focused on activation of the cardiac sympathetic nervous system, is simple to implement, is easy to use and is comparatively cost effective.

### SUMMARY OF THE INVENTION

In accordance with the present invention, a system and method for electrically stimulating a sympathetic nerve to improve heart function employs an electrode that is positioned in the vasculature of a patient for epicardial heart stimulation. More particularly, the electrode is positioned in the coronary sinus, or in a vein connected to the coronary sinus. And, it is positioned adjacent to a sympathetic nerve (i.e. nerve bundle). Selective stimulation of the sympathetic nerve by the electrode then causes a secretion (release) of norepinephrine that improves the heart muscle contraction (i.e. left ventricle) in the area of the electrode, blood vessel (vein) and nerve.

Structurally, the system of the present invention, includes a voltage source and a sensor that are respectively connected to a deployment catheter. Specifically, the voltage source is electrically connected to an electrode that is located at/near the distal end of the deployment catheter. Similarly, the sensor is electrically connected to a probe that is also located at/near the end of the deployment catheter. Further, the system can include a computer having pre-programmed instructions for controlling the operation of the voltage source. As envisioned for the present invention, the voltage source can be either a pacemaker or a pacing catheter of a type well known in the pertinent art.

For an operation of the present invention, an appropriate sympathetic nerve (nerve bundle) on the epicardial surface of the left ventricle is identified. Importantly, the nerve needs to be located either adjacent the coronary sinus or a vein that is connected to the coronary sinus. Location of this nerve can be accomplished by well known mapping techniques, such as by using a loop catheter. Once the site of an appropriate sympathetic nerve has been identified, the electrode is advanced through the venous system to be positioned at the site adjacent to the nerve in the vein. The electrode can then be activated.

Activation of the electrode can be accomplished either actively, in accordance with pre-programmed instructions from the computer, or reactively in response to the contractions of the heart muscle. In either case, an activation of the electrode to stimulate the sympathetic nerve for a release of norepinephrine is preferably accomplished while the heart is in a refractory period. Stated differently, the sympathetic nerve should be stimulated while the heart muscle is not able to electrically respond to electrical stimuli. The released norepinephrine, however, is available for stimulation of the heart muscle when the heart is ready for its next contraction. Thus, stimulation of the sympathetic nerve only indirectly causes a heart muscle contraction. Further, the voltage level necessary for stimulating the sympathetic nerve is lower than the threshold necessary for a direct stimulation of the heart muscle.

As envisioned for the present invention, and as implied above, the system of the present invention may operate in any of several modes. For one, the computer can be pre-programmed with low-intensity stimulation patterns. For another, the sensor can be used to identify when electrical activation of the heart muscle occurs, and can then activate the voltage source for stimulation of the nerve during the heart's refractory period. Still another mode would be to couple the system into a larger assembly for the execution of other stimulation plans.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a schematic drawing of the components of a system for the present invention;
Fig. 2 is a drawing of the diaphragmatic surface of the heart showing only veins and proximate nerve bundles; and
Fig. 3 is a time graph of the electrical and mechanical cycles of the heart.

### DESCRIPTION OF THE PREFERRED EMBODIMENTS

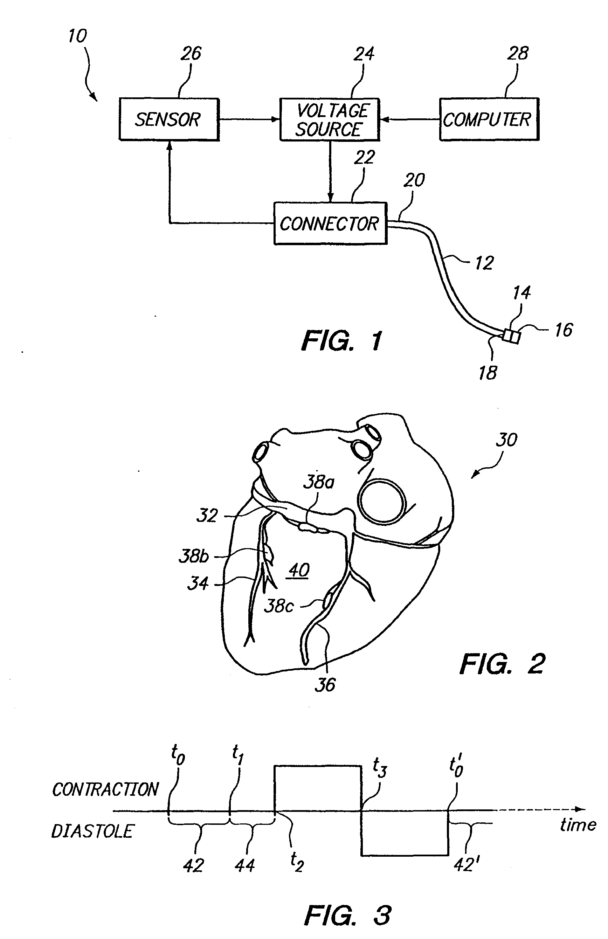

Referring initially to Fig. 1, a system for transvascular activation of sympathetic cardiac nerves that are useful for improving heart function in accordance with the present invention is shown and is generally designated 10. As shown, the system 10 includes a deployment catheter 12 having an electrical probe 14 and an electrode 16 mounted at its distal end 18. The proximal end 20 of the deployment catheter 12 is affixed to an electrical connector 22. As also shown in Fig. 1, the system 10 includes a voltage source 24 that is electrically connected to the connector 22. For purposes of the present invention, the voltage source 24 may be a pacemaker or a pacing catheter of a type well known in the pertinent art.

Still referring to Fig. 1, it will be seen that the system 10 further includes a sensor 26 and a computer 28 that are each electrically connected to the voltage source 24. Also, the voltage source 24 is connected, through the connector 22, with the electrode 16 that is mounted at the distal end 18 of the deployment catheter 12. Further, the sensor 26 is connected with the probe 14 through the connector 22. With these connections, it is to be appreciated that the voltage source 24 is responsive to both the sensor 26 and to the computer 28.

A heart muscle is shown in Fig. 2 and is generally designated 30. Anatomically, a view of the diaphragmatic surface of the heart muscle 30, (Fig. 2), shows its coronary sinus 32 and several connecting veins. In particular, the posterior vein 34 of the left ventricle, and the middle cardiac vein 36 are shown. Also shown are sympathetic nerve(s) 38 in the nervous system, of which the nerve bundles 38a, 38b and 38c are only exemplary. Importantly, the nerves 38 are located on the epicardial surface of the left ventricle 40, and they are adjacent to either the coronary sinus 32 or one of the veins connected with the coronary sinus 32 (e.g. veins 34 or 36).

For the operation of the system 10 of the present invention, the heart muscle 30 is initially mapped to identify an appropriate nerve(s) 38. Specifically, as implied above, an appropriate nerve 38 is one that is adjacent the coronary sinus 32 or a vein that is connected with the coronary sinus 32 (e.g. vein 34 or 36). Also, it is important that the nerve 38 be at a location on the epicardial surface of the left ventricle 40 of heart muscle 30 where it will be efficacious for stimulating the heart muscle 30. Once an appropriate nerve(s) 38 has been identified, the deployment catheter 12 is advanced through the vasculature of the patient (not shown) to position the electrode 16 in the vein (e.g. 32, 34 or 36) for stimulation of the adjacent nerve (e.g. respectively 38a, 38b or 38c).

Fig. 3 provides a generalized time graph for the cyclical activity of the heart muscle 30. More specifically, Fig. 3 provides a sequential time line for the interaction between the system 10 and the heart muscle 30 during a beat of the heart muscle 30. This includes, an electrical activation of system 10, the subsequent mechanical activation of the heart muscle 30 (i.e. contraction), and the subsequent relaxation or diastole that follows each contraction. The cycle, of course, is repetitive.

As shown for a single beat of the heart muscle 30, activation of the heart muscle 30 begins at the time "t₀". This activation continues from the time "t₀" to the time "t₁", through what is known as the refractory period 42. In more detail, the refractory period 42 lasts for a time duration of about 120 to 300 ms, and occurs when the heart muscle 30 is not able to respond to an electrical stimulation. The nerve 38, however, is able to respond during a refractory period 42 by secreting norepinephrine. As envisioned for the system 10 of the present invention, the magnitude of the electric pulse that is provided by voltage source 24 for use in stimulating the nerve 38 during the refractory period 42 can be controller. Specifically it can be programmed to be less than a level that would otherwise be efficacious for directly stimulating a contraction of the heart muscle 30.

At the time "t₁" shown in Fig. 3, the refractory period 42 ends and a relative refractory period 44 begins wherein the heart muscle 30 electrically recovers from the refractory period 42. The relative refractory period 44 then ends at the time "t₂" when the heart muscle 30 contracts. Importantly, this contraction is assisted by the norepinephrine that was secreted by nerve 38 in response to an activation of the voltage source 24 during the refractory period 42. As shown, this contraction is followed by a relaxation or diastole that lasts from a time "t₃" until another cycle begins at the time "t₀' ". For purposes of the present invention, the computer 28 can be pre-programmed to accomplish the described cycle. Alternatively, the sensor 26 can receive a signal from the probe 14 that indicates a spontaneous electrical activation signal, and the voltage source 24 can then be responsive to the sensor 26 by activating the electrode 16 during the respective refractory period.

Several characteristics of the electrical pulses from the voltage source 24 are important for the present invention. For one, the magnitude and duration of each pulse can be pre-programmed and, thus, varied as required for the particular patient's needs. For another, the magnitude of each pulse should be and, indeed, is preferably below the voltage threshold that would otherwise be required to directly stimulate the heart muscle 30. With these considerations in mind, low-intensity stimulation patterns can be crafted to meet specific patient needs.

While the particular System and Method for Transvascular Activation of Cardiac Nerves to Improve Heart Function as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for electrically stimulating a sympathetic nerve to improve heart function which comprises:
a means for identifying a site in the coronary sinus and connected veins of a patient, wherein the site is adjacent the nerve;
an electrode positioned at the site for electrically stimulating the nerve; and
a means for activating the electrode to release norepinephrine from the sympathetic nerve for improved heart muscle contraction.

2. A system as recited in claim 1 wherein the activating means is a voltage source for selectively transmitting electric pulses to the electrode in accordance with a predetermined program, wherein the program establishes a duration and a magnitude for each electric pulse and wherein the magnitude of each electric pulse can be programmed to be less than an effective level for directly stimulating a heart muscle contraction.

3. A system as recited in claim 1 wherein the voltage source is a pacemaker or a pacing catheter.

4. A system as recited in claim 1 wherein the identifying means comprises a loop catheter for selecting the site for neural stimulation.

5. A system as recited in claim 1 wherein the electrode is activated during a refractory period of heart muscle function to avoid an inadvertent direct stimulation of the heart muscle.

6. A system as recited in claim 1 further comprising a means for sensing activation of the heart muscle, wherein the sensing means is connected to the activating means for activation thereof.

7. A system as recited in claim 1 wherein the activating means includes a programmable means for allowing an intermittent application of stimulation.

8. A system for electrically stimulating a sympathetic nerve to improve heart function which comprises:
a deployment catheter having a proximal end and a distal end;
an electrode mounted on the distal end of the deployment catheter to be positioned at a site in the venous system of a patient adjacent to the sympathetic nerve, wherein the sympathetic nerve is located on an epicardial surface of the heart; and
a voltage source connected to the proximal end of the deployment catheter for selectively transmitting electrical pulses to activate the electrode during a refractory period in a diastole phase of the heart to release norepinephrine from the sympathetic nerve for improved heart muscle contraction.

9. A system as recited in claim 8 further comprising:
a computer electronically connected to the voltage source for activating the electrode in accordance with a pre-programmed low-intensity stimulation pattern;
a probe mounted at the distal end of the deployment catheter for determining when the heart muscle experiences a contraction; and
a sensor connected to the probe, and to the voltage source, for activating the electrode in response to a contraction of the heart muscle as determined by the probe.

10. A system as recited in claim 8 wherein the magnitude of each electric pulse is less than an effective level for directly stimulating a heart muscle contraction.

11. A system as recited in claim 10 wherein the voltage source is a pacemaker or a pacing catheter.

12. A method for stimulating a sympathetic nerve to improve heart function which comprises the steps of:
identifying a site in the coronary sinus and connected veins of a patient, wherein the site is adjacent the nerve;
positioning an electrode at the site; and
activating the electrode to electrically stimulate the sympathetic nerve for release of norepinephrine therefrom to improve heart muscle contraction.

13. A method as recited in claim 12 wherein the activating step is accomplished during a refractory period in a diastole phase of the heart.

14. A method as recited in claim 12 wherein the electrode is activated in accordance with a pre-programmed low-intensity stimulation pattern.

15. A method as recited in claim 12 wherein the electrode is activated in response to a contraction of the heart muscle.
